(19) Europäisches Patentamt
European Patent Office
Office européen des brevets

(11) **EP 4 175 704 B1**

(12) **EUROPÄISCHE PATENTSCHRIFT**

(45) Veröffentlichungstag und Bekanntmachung des Hinweises auf die Patenterteilung:
**14.05.2025 Patentblatt 2025/20**

(21) Anmeldenummer: **21723125.7**

(22) Anmeldetag: **21.04.2021**

(51) Internationale Patentklassifikation (IPC):
*A61M 16/00* (2006.01)     *A61M 16/10* (2006.01)
*F04D 33/00* (2006.01)     *F04C 18/44* (2006.01)
*F04C 18/356* (2006.01)     *F04C 18/12* (2006.01)
*F04C 29/00* (2006.01)     *F04C 29/06* (2006.01)
*F04C 23/00* (2006.01)

(52) Gemeinsame Patentklassifikation (CPC):
**A61M 16/0057; F04C 18/3562; F04C 23/001; F04C 29/0035;** A61M 16/0072; A61M 2205/07; A61M 2205/42; F04C 2250/20; F04C 2250/301; F04C 2270/14; F04C 2270/20

(86) Internationale Anmeldenummer:
**PCT/DE2021/100368**

(87) Internationale Veröffentlichungsnummer:
**WO 2022/002291 (06.01.2022 Gazette 2022/01)**

(54) **VORRICHTUNG ZUR BEATMUNG SOWIE VERFAHREN ZUR BEREITSTELLUNG EINES ATEMGASES**

RESPIRATORY DEVICE AND METHOD FOR PROVIDING A RESPIRATORY GAS

DISPOSITIF RESPIRATOIRE ET PROCÉDÉ POUR FOURNIR UN GAZ RESPIRATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.07.2020 DE 102020117343**

(43) Veröffentlichungstag der Anmeldung:
**10.05.2023 Patentblatt 2023/19**

(73) Patentinhaber: **Weinmann Emergency Medical Technology GmbH + Co. KG**
**22525 Hamburg (DE)**

(72) Erfinder: **HERRMANN, Frank**
**25355 Barmstedt (DE)**

(74) Vertreter: **Klickow & Wetzel PartGmbB**
**Jessenstraße 4**
**22767 Hamburg (DE)**

(56) Entgegenhaltungen:
EP-A1- 1 486 677      EP-A1- 3 597 923
EP-B1- 1 780 478      US-A1- 2011 058 970
US-A1- 2011 247 620    US-A1- 2014 219 845

**Beschreibung**

Die Offenbarung betrifft eine Pumpvorrichtung im Sinne einer Verdrängerpumpe, insbesondere ausgebildet zur Förderung von Gasen.

Darüber hinaus betrifft die Offenbarung eine Vorrichtung zur Beatmung aufweisend eine derartige Pumpvorrichtung.

Weiterhin betrifft die Offenbarung ein Verfahren zur Bereitstellung von Atemgas mithilfe einer Vorrichtung zur Beatmung.

Bei der maschinellen Beatmung von Patienten mithilfe von Vorrichtungen zur Beatmung bestehen für eine effektive Umsetzung der Beatmung diverse spezielle Anforderungen.

In extremen Beatmungssituationen, beispielsweise bei einem Minutenvolumen $V = 10$ $L/min$ und einer maximale Druckerhöhung $\Delta p = 60$ $hPa$, beträgt die mittlere pneumatische Leistung $P_{mittel} = V * \Delta p = 1$ W, womit der pneumatische Leistungsbedarf gering ist. Die Anforderungen verschärfen sich durch einen intermittierenden Betrieb, bei dem eine kurze Inspiration mit schnellen Druckanstiegen und hohen Flows (Volumenströmen) einhergeht. Die dabei notwendigen Spitzenflows von bis zu $V = 180$ $L/min$ und Druckerhöhungen von bis zu $\dot{\Delta p} = 180$ $hPa/s$ erfordern zwar höhere pneumatische Spitzenleistungen, die mittlere pneumatische Leistung bleibt jedoch in einem niedrigen Bereich von wenigen Watt.

Bekannte Vorrichtungen zur Beatmung weisen zur Erzeugung der benötigten Flows und Drücke in der Regel Gebläse auf, die mithilfe eines in einem Gehäuse rotierenden Lüfterrads die Luft über einen Einlass ansaugen und durch einen Auslass fördern. Derartige Gebläse bieten diverse Vorteile wie einen kompakten und einfachen Aufbau, eine hohe Flowleistung und eine hohe Dynamik und stellen darüber eine nahezu ideale Druckquelle dar. Jedoch ist zum Druckaufbau ein großer Drehzahlhub notwendig, die Eignung als Flowquelle ist eher gering, die stark turbulente Strömung des austretenden Fluids erschwert eine nachgeschaltete Flowmessung und der mittlere pneumatische Gesamtwirkungsgrad liegt deutlich unter 10 %. Im Detail führen schnelle Drehzahlanstiege mit einhergehenden Druckanstiegen zu einer deutlichen Änderung der im Lüfterrad gespeicherten Rotationsenergie, wofür hohe Leistungen erforderlich sind. Diese benötigten Leistungen liegen in einem Bereich über 40 W und können bei sehr schnellen benötigten Anstiegen des Drucks deutlich höher sein. Bei hoher Drehzahl besteht darüber hinaus ein hoher statischer Leistungsbedarf. Insgesamt entsteht bei der Verwendung von Gebläsen in Vorrichtungen zur Beatmung dadurch sehr viel Wärme, die abgeführt werden muss, sodass Kühlmaßnahmen unabdingbar sind.

Der Stand der Technik weist als Alternativen zum Gebläse insbesondere Kolbenpumpen und Membranpumpen zur Verwendung in Vorrichtungen zur Beatmung auf, die zwar teilweise geringere Turbulenzen des ausströmenden Fluids und weniger Verlustwärme ermöglichen, jedoch aufgrund ihrer Bauart besonders hohe Anforderungen an die Bauteiltoleranzen stellen, sodass die Produktionskosten für eine entsprechend genaue Dimensionierung der Bauteile erhöht sind.

Eine Aufgabe der Offenbarung ist es, eine Vorrichtung zur Beatmung mit einer Pumpvorrichtung anzugeben, die im Vergleich mit einem Gebläse verbesserte Eigenschaften bezüglich der Leistungsaufnahme und der Wärmeabgabe aufweist.

Eine Aufgabe der Erfindung ist es, eine Vorrichtung zur Beatmung mit einer Pumpvorrichtung anzugeben, die im Vergleich mit einem Gebläse geringere Turbulenzen des austretenden Fluids ermöglicht.

Diese Aufgabe wird erfindungsgemäß durch eine Vorrichtung zur Beatmung gemäß Patentanspruch 1 und durch ein Vefahren gemäß Anspruch 11 gelöst.

Eine weitere Aufgabe der Offenbarung ist es, eine Vorrichtung zur Beatmung mit einer Pumpvorrichtung anzugeben, die geringere Anforderungen an die Bauteiltoleranzen stellt.

In den Unteransprüchen sind vorteilhafte Ausprägungen der Erfindung angegeben.

Die nachfolgend offenbarten Merkmale einer Pumpvorrichtung sind sowohl einzeln als auch in allen ausführbaren Kombinationen Teil der Erfindung.

Eine offenbarungsgemäße Pumpvorrichtung ist als eine Verdrängerpumpe ausgebildet und weist mindestens eine Pumpkammer und je Pumpkammer einen in dieser angeordneten Läufer auf.

Die Pumpkammer ist in einem Gehäuse angeordnet und ist als Stator der Pumpvorrichtung ausgebildet.

Die Pumpkammer weist einen Einlass und einen Auslass auf, durch die ein Fluid in die Pumpkammer einströmen bzw. ausströmen kann.

Der Läufer ist innerhalb der Pumpkammer an der Innenwand der Pumpkammer entlang geführt beweglich.

In einer Ausführungsform der Offenbarung ist der Läufer rotationsfrei innerhalb der Pumpkammer beweglich, indem Sinne, dass zwar die Position des Läufers innerhalb der Pumpkammer, jedoch nicht dessen Orientierung veränderbar ist.

In einer Ausführungsform der Erfindung ist der Läufer bzw. ein Punkt des Läufers auf einer Orbitalbahn geführt beweglich.

In einer Ausführungsform der Offenbarung ist der Läufer auf einer rotierbaren Welle eines Antriebes exzentrisch und mithilfe einer Entkoppeleinrichtung von der Rotation entkoppelt gelagert, sodass eine Orbitalbewegung des Läufers umsetzbar ist.

In einer Ausführungsform der Offenbarung ist die Entkoppeleinrichtung als ein Wälzlager ausgebildet.

Der Läufer ist innerhalb der Pumpkammer derart bewegbar, dass in der Pumpkammer in Bewegungsrichtung hinter dem Läufer ein Saugraum ausbildbar ist, in den durch die Bewegung des Läufers über den Einlass ein Fluid ansaugbar ist. In Bewegungsrichtung vor dem Läufer ist innerhalb der Pumpkammer ein Druckraum ausbildbar, aus dem das Fluid durch die Bewegung des Läufers über den Auslass aus der Pumpkammer heraus förderbar ist.

Als Saugraum ist dabei in Abhängigkeit der Winkelposition des Läufers in der Pumpkammer abzüglich des Volumens des Läufers dasjenige Volumen der Pumpkammer definiert, das eine Verbindung zum Einlass aufweist und als Druckraum ist abzüglich des Volumens des Läufers dasjenige Volumen der Pumpkammer definiert, dass keine Verbindung zum Einlass aufweist.

In einer Ausführungsform der Offenbarung sind der Einlass und der Auslass der Pumpkammer an einem gemeinsamen, mit der Pumpkammer verbundenen Kanal angeordnet. Der Kanal ist mithilfe eines Trennelements in einen dem Einlass und einen dem Auslass zugordneten Bereich getrennt.

In einer Ausführungsform der Offenbarung ist das Trennelement mit dem Läufer verbunden.

In einer Ausführungsform der Offenbarung ist das Trennelement als ein zumindest bereichsweise stielartig geformter Pleuel ausgebildet, der mit dem Läufer verbunden ist.

In einer bevorzugten Ausführungsform der Offenbarung ragt der mit dem Läufer verbundenen Pleuel aus der Pumpkammer in den Bereich des Einlasses und des Auslasses der Pumpkammer und dichtet den Einlass gegenüber dem Auslass ab.

In einer Ausführungsform der Offenbarung ist der Pleuel des Läufers an dem dem übrigen Läufer angewandten Ende linear geführt.

In einer Ausführungsform der Offenbarung ist der Stator, insbesondere die Wand der Pumpkammer aus einem Metall gefertigt, sodass eine hohe Formstabilität und Wärmeleitfähigkeit gegeben sind.

In einer bevorzugten Ausführungsform der Offenbarung ist die Wand der Pumpkammer aus Aluminium ausgebildet, das neben einer hohen Formstabilität und Wärmeleitfähigkeit eine relativ geringe Masse aufweist.

In einer Ausführungsform der Offenbarung ist der Läufer aus einem Gleitkunststoff ausgebildet, sodass eine geringe Reibung zwischen Läufer und Innenwand der Pumpkammer realisiert ist.

Der Verlauf des Flows des aus dem Auslass der Pumpkammer strömenden Fluids ist dabei unmittelbar gekoppelt an den Verlauf des Volumens des Saug- bzw. des Druckraumes der Pumpvorrichtung in Abhängigkeit der Winkelposition des Läufers innerhalb der Pumpkammer.

In einer bevorzugten Ausführungsform der Offenbarung weist der Läufer mindestens einen Hohlraum zur Verringerung der Masse des Läufers auf. Je geringer die Masse des Läufers ist, desto geringer ist die für eine Anpassung der Laufgeschwindigkeit des Läufers erforderliche Energie.

In einer bevorzugten Ausführungsform der Offenbarung ist der Läufer in Relation zur Innenwand der Pumpkammer so ausgebildet, dass dieser auf der mithilfe des Exzenterantriebs realisierten orbitalen Bewegungsbahn des Läufers in genau einer Position auf der Bewegungsbahn im Querschnitt zwei Berührungspunkte mit der Innenwand der Pumpkammer aufweist, während der Läufer auf allen anderen Positionen im Querschnitt nur einen Berührungspunkt mit der Innenwand der Pumpkammer aufweist.

Je nach dreidimensionaler Ausbildung des Läufers und der Pumpkammer ist insgesamt eine Berührungslinie bzw. sind zwei Berührungslinien je nach Position des Läufers realisiert. Bei einem scheibenartigen Aufbau, bei dem sich der Läufer und die Pumpkammer jeweils mit einer konstant geformten Fläche über eine Höhe h ausdehnen, sind die Berührungslinien Geraden bzw. Abschnitte von Geraden.

In einer bevorzugten Ausführungsform der Offenbarung ist das Trennelement in Umfangsrichtung des Läufers zwischen den beiden Berührungspunkten von Läufer und Innenwand der Pumpkammer angeordnet, wobei in Umfangsrichtung der geringere Abstand der Berührungspunkte maßgeblich ist.

In einer besonders bevorzugten Ausführungsform der Offenbarung sind in derjenigen Position, in der der Läufer zwei Berührungspunkte mit der Innenwand der Pumpkammer aufweist, sowohl der Weg vom Einlass als auch der Weg vom Auslass in das übrige Volumen der Pumpkammer versperrt, sodass ein Volumen innerhalb der Pumpkammer eingeschlossen ist.

Die Position, in der der Läufer und die Innenwand der Pumpkammer zwei Berührungspunkte aufweisen sei als Nullposition oder 0° Winkelposition in Bezug auf die orbitale Bewegungsbahn definiert. Der Winkel steigt je nach Position des Läufers in dessen Bewegungsrichtung an, bis bei der Winkelposition von 360° die Nullposition wieder erreicht ist.

In einer Ausführungsform der Offenbarung ist im Bereich des Läufers mindestens eine Feder angeordnet, mithilfe der der Läufer an die Innenwand der Pumpkammer andrückbar ist, sodass eine Abdichtung an dem mindestens einen Berührungspunkt zwischen Läufer und Innenwand der Pumpkammer verbessert ist. Dadurch sind insbesondere höhere Fertigungstoleranzen ermöglicht.

In einer anderen Ausführungsform der Offenbarung ist die Gehäusewand so gestaltet, dass die Gehäusewand federnd gegen einen fest gelagerten Läufer ausgebildet ist, sodass insbesondere höhere Fertigungstoleranzen ermöglicht sind.

In einer Ausführungsform der Offenbarung ist die Gehäusewand der Pumpkammer etwas kleiner als die ideale Kontur für den korrespondierenden Läufer ausgebildet und vom Läufer am Berührungspunkt / an den Berührungspunkten geringfügig nach außen drückbar.

In einer weiteren Ausführungsform der Offenbarung weist das Gehäuse der Pumpkammer nach innen in diese hineinragende Lamellen auf, die sich quer zur Bewegungsrichtung des Läufers in der Pumpkammer erstrecken und die den Läufer im Bereich des Berührungspunktes bzw. der Berührungspunkte kontaktieren. Der in der Pumpkammer entstehende Druck drückt die Lamellen dabei an den Läufer an, wobei der Anpressdruck der Lamellen ohne nennenswerten Ausgangsdruck sehr gering ist.

In einer weiteren Ausführungsform der Offenbarung sind eine federnde Lagerung des Läufers und eine der vorstehend beschriebenen Ausgestaltungen des Gehäuses der Pumpkammer miteinander kombiniert.

In einer bevorzugten Ausführungsform der Offenbarung sind die Form der Pumpkammer und des Läufers derart aufeinander abgestimmt, dass bei einer konstanten Geschwindigkeit des Läufers ein sprungfreier Verlauf des Flows des geförderten Fluids am Auslass der Pumpkammer realisiert ist.

In einer bevorzugten Ausführungsform der Offenbarung sind die Form der Pumpkammer und des Läufers derart aufeinander abgestimmt, dass bei einer konstanten Geschwindigkeit des Läufers ein etwa sinusförmiger Verlauf des Flows des geförderten Fluids am Auslass der Pumpkammer realisiert ist, wobei das Minimum des Flows etwa bei 0 liegt (kein Flow in die entgegengesetzte Richtung).

In einer Ausführungsform der Offenbarung ist der Läufer kreisförmig ausgebildet. Aufgrund der Stärke der Trennwand zwischen Einlass und Auslass, in Ausführungsformen der Erfindung realisiert durch das beispielsweise als ein Pleuel ausgebildete Trennelement, ist der Verlauf des Flows des Fluids am Auslass jedoch leicht abweichend von einer Sinuskurve ausgebildet.

Je dünner das Trennelement, bzw. der Stiel des Pleuels ist, desto näher liegt der Verlauf des Flows des Fluids am Auslass an einer Sinusform. In Ausführungsformen der Erfindung ist das Trennelement oder der Pleuel daher nicht starr, sondern als eine dünne Trennschicht ausgebildet, die an dem dem Läufer abgewandten Ende den Einlass vom Auslass abdichtend mit dem Gehäuse der Pumpvorrichtung verbunden ist.

Dabei ist das Trennelement bzw. der Pleuel und/oder die dichtende Verbindung von Trennelement bzw. Pleuel und Gehäuse flexibel ausgebildet, sodass die Bewegung des Läufers auf der Orbitalbahn nicht gestört ist.

Das mit der Pumpvorrichtung in einem Umlauf des Läufers förderbare Volumen ist abhängig von der Exzentrität des Läufers, wobei das Pumpvolumen mit steigender Exzentrität steigt. Dies ist dadurch begründet, dass die Obritalbahn des Läufers bei steigender Exzentrität verlängert wird, sodass auch die Größe der Pumpkammer entsprechend angepasst werden muss.

Ein Nachteil eines kreisförmigen Läufers ist, dass für einen möglichst sinusförmigen Verlauf des Flows eines aus dem Auslass ausströmenden Fluids nur eine geringe Exzentrität möglich ist, sodass nur ein geringes Pumpvolumen realisierbar ist. Zur Adressierung dieser Probleme ist die Form des Läufers in bevorzugten Ausführungsformen der Erfindung vom Kreis weg angepasst.

In einer bevorzugten Ausführungsform der Offenbarung weist der Läufer eine kreisförmige Grundform auf, die um jeweils seitlich angrenzend zum Trennelement angeordnete und teilweise über die kreisförmige Grundform des Läufers hinausragende Kreise mit einem kleineren Durchmesser als die kreisförmige Grundform erweitert ist. Die Übergänge zwischen der Grundform und den kleineren Kreisen ist dabei zumindest auf den dem Trennelement abgewandten Seiten fließend ausgeführt.

In einer weiteren Ausführungsform der Offenbarung weist der Läufer entsprechend der vorstehenden Ausführungsform anstatt eines Kreises als Grundform eine elliptische Grundform auf, wobei die dem Trennelement zugewandte Hälfte der Ellipse durch zwei sich bereichsweise überschneidende Kreise modifiziert ist und der Übergang der Ellipse in die Kreise sanft gestaltet ist.

In einer anderen Ausführungsform der Offenbarung ist die Form des Läufers entsprechend zu einer Spline-Funktion ausgebildet.

Spline-Funktionen sind zur Ausformung des Läufers vorteilhaft einsetzbar, da sie einen Kurvenverlauf ohne Sprünge ermöglichen. Durch die Generierung einer Spline-Funktion in kartesischen Koordinaten und deren Übertragung in Polar-Koordinaten (Radius über Winkel) sind beliebig viele, sprungfreie Läuferkurven bildbar, die hinsichtlich des Flowverlaufs weitergehend optimierbar sind.

Der etwa sinusförmige Verlauf des ausströmenden Fluids ist in einer Ausführungsform der Erfindung realisiert durch einen etwa sinusförmig verlaufenden freien Querschnitt im Bereich des Auslasses bzw. der Verbindung des Auslasses mit der Pumpkammer während des Umlaufes des Läufers in der Pumpkammer.

Die Form des Läufers und die Form der Pumpkammer sind in bevorzugten Ausführungsformen der Erfindung in 0° und 180° Winkelposition des Läufers symmetrisch bezüglich der die Winkel 0°-180° verbindenden Achse.

In einer bevorzugten Ausführungsform der Offenbarung weist die Pumpvorrichtung zwei in Bezug auf die Winkelposition auf ihren Orbitalbahnen um 180° phasenverschoben laufende Läufer in jeweils einer Pumpkammer für einen insgesamt nahezu konstanten Volumenstrom des Fluids an einem gemeinsamen Auslass auf.

Dies ist in einer vorteilhaften Ausführungsform der Offenbarung durch die Überlagerung zweier um 180° phasenverschobener etwa sinusförmiger Flowverläufe der einzelnen Pumpkammer-Läufer-Anordnungen realisiert.

In einer bevorzugten Ausführungsform der Offenbarung sind die Läufer auf einer gemeinsamen Welle exzentrisch antreibbar, sodass ein sehr guter Massenausgleich und ein nahezu pulsationsfreier Gesamtflow ermöglicht sind.

Durch den Massenausgleich werden zudem die Vibrationen der Pumpvorrichtung reduziert.

Eine offenbarungsgemäße Vorrichtung zur Beatmung weist zur Förderung des Atemgases mindestens eine Pumpvorrichtung entsprechend der vorstehenden Beschreibung auf.

In einer bevorzugten Ausführungsform einer offenbarungsgemäßen Vorrichtung zur Beatmung weist diese eine Pumpvorrichtung mit zwei in Bezug auf ihre Winkelposition um 180° versetzt ausgerichteten Läufern in jeweils einer Pumpkammer auf, mit denen ein nahezu pulsationsfreier Verlauf des Flows des Atemgases an einem gemeinsamen Auslass realisiert ist.

In einer Ausführungsform einer offenbarungsgemäßen Vorrichtung zur Beatmung weist diese weiterhin eine Flowmesseinrichtung zur Messung des Volumenstromes des Atemgases auf.

In einer bevorzugten Ausführungsform einer offenbarungsgemäßen Vorrichtung zur Beatmung weist diese eine Regelvorrichtung zur Regelung des Flows des Atemgases am Auslass der Pumpvorrichtung auf. Insbesondere ist dazu die Drehzahl des Exzenterantriebes des mindestens einen Läufers der Pumpvorrichtung mithilfe einer Steuereinheit steuerbar.

Die im Folgenden offenbarten Merkmale eines Verfahrens zur Bereitstellung von Atemgas sind sowohl einzeln als auch in allen ausführbaren Kombinationen Teil der Erfindung.

Ein offenbarungsgemäßes Verfahren zur Bereitstellung von Atemgas weist zumindest die folgenden Verfahrensschritte auf:

Verschieben eines Läufers in einer Pumpkammer aus einer 0° Winkelposition, in der der Läufer zwei Berührungspunkte mit der Innenwand der Pumpkammer aufweist und dadurch den Einlass- und den Auslassbereich gegenüber der übrigen Pumpkammer (Druckraum) abdichtet, in Bewegungsrichtung des Läufers auf einer Orbitalbahn in eine Position, in der der Läufer und die Innenwand der Pumpkammer genau einen Berührungspunkt aufweisen, Trennen eines Druckraumes in Bewegungsrichtung vor dem Läufer und eines Saugraumes in Bewegungsrichtung hinter dem Läufer durch den Berührungspunkt des Läufers und der Innenwand der Pumpkammer, Ansaugen von Atemgas vom Einlass in den Saugraum und Fördern des Atemgases aus dem Druckraum über den Auslass durch eine kontinuierliche Verschiebung des Läufers in der Pumpkammer, wobei mit einem zweiten Läufer in einer zweiten Pumpkammer die gleichen Verfahrensschritte in einem 180° phasenversetzten Ablauf umgesetzt werden, sodass bei einer auf den jeweiligen Orbitalbahnen umlaufenden Bewegung der Läufer innerhalb der jeweiligen Pumpkammer bei einer konstanten Laufgeschwindigkeit an einem gemeinsamen Auslass ein möglichst sprungfreier Flowverlauf des Atemgases realisiert wird.

In einer bevorzugten Ausführungsform des offenbarungsgemäßen Verfahrens wird der Flow des Atemgases durch eine Steuerung der Bewegungsgeschwindigkeit der Läufer geregelt.

In einer vorteilhaften Ausführungsform des offenbarungsgemäßen Verfahrens wird eine offenbarungsgemäße Pumpvorrichtung oder eine offenbarungsgemäße Vorrichtung zur Beatmung aufweisend eine offenbarungsgemäße Pumpvorrichtung entsprechend der vorstehenden Beschreibung verwendet.

In vorteilhaften Ausführungsformen weist die Erfindung zumindest die folgenden Vorteile und Eigenschaften auf:

- Eine geringe (Massen-)Trägheit im System für energiearme Druck-/Flow-Änderung
- Ein geringer Leistungsbedarf bei konstantem Druck oder Flow
- Die Möglichkeit der Druck- und/oder Flow-kontrollierten Beatmung
- Eine kompakte Bauweise mit niedrigem Gewicht
- Eine realisierbare Druckerhöhung von mindestens $\Delta p = 60\ hPa$
- Einen realisierbaren Druckanstieg von $\dot{\Delta}p = 180\ hPa/s$
- Einen Spitzenflow von $V = 180$ $\dot{V} = 180\ \frac{L}{min}$

Durch die niedrige Rotationsenergie im offenbarungsgemäßen System ist ein hoch dynamisches und zugleich energiesparendes System ermöglicht. Im Idealfall ist ein Halten des Drucks ohne Bewegung des Läufers oder der Läufer möglich. Die Anforderungen an die Systemkühlung sind geringer als beim Gebläse, da der größte Energiebedarf für die Änderung der Rotationsenergie benötigt wird, die in einem offenbarungsgemäßen System geringer ist. Im Gegensatz zum Gebläse ist die Strömung des ausströmenden Fluids weniger turbulent, wodurch Vorteile für eine nachgeschaltete Flowmessung gegeben sind.

Das vorstehend beschriebene erfindungsgemäße Konstruktionsprinzip kann optional auch für Kompressoren oder für Vakuumpumpen angewendet werden.

In den Figuren sind beispielhafte Ausführungsformen der Offenbarung dargestellt. Es zeigen:

Figur 1: Einen Querschnitt einer erfindungsgemäßen Pumpvorrichtung im Bereich der Pumpkammer in der 0° Winkelposition des Läufers,

Figur 2: Einen Querschnitt einer erfindungsgemäßen Pumpvorrichtung im Bereich der Pumpkammer in der 60° Winkelposition des Läufers,

Figur 3: Einen Querschnitt einer erfindungsgemäßen Pumpvorrichtung im Bereich der Pumpkammer in der 180° Winkelposition des Läufers,

Figur 4:    Einen Querschnitt einer erfindungsgemäßen Pumpvorrichtung im Bereich der Pumpkammer in der 300° Winkelposition des Läufers,

Figur 5:    Ein Diagramm der Flowverläufe des Fluids am Auslass zweier Pumpkammern und einem gemeinsamen Auslass beider Pumpkammern,

Figur 6:    Eine perspektivische Ansicht eines Querschnitts einer offenbarungsgemäßen Pumpvorrichtung,

Figur 7:    Eine perspektivische Ansicht eines Schnitts in Längsrichtung einer erfindungsgemäßen Pumpvorrichtung mit zwei Pumpkammern und zwei Läufern,

Figur 8:    Eine schematische Darstellung des Querschnitts eines Läufers einer offenbarungsgemäßen Vorrichtung,

Figur 9:    Den radialen Verlauf des Radius r des in der Figur 8 dargestellten Läufers über dem Winkel $\alpha$,

Figur 10:    Eine schematische Darstellung des Querschnitts eines Läufers in einer Pumpkammer einer offenbarungsgemäßen Vorrichtung,

Figur 11:    Einen Querschnitt einer weiteren offenbarungsgemäßen Ausführungsform einer Pumpvorrichtung und

Figur 12:    Ein Ablaufdiagramm eines offenbarungsgemäßen Verfahrens zur Bereitstellung von Atemgas.

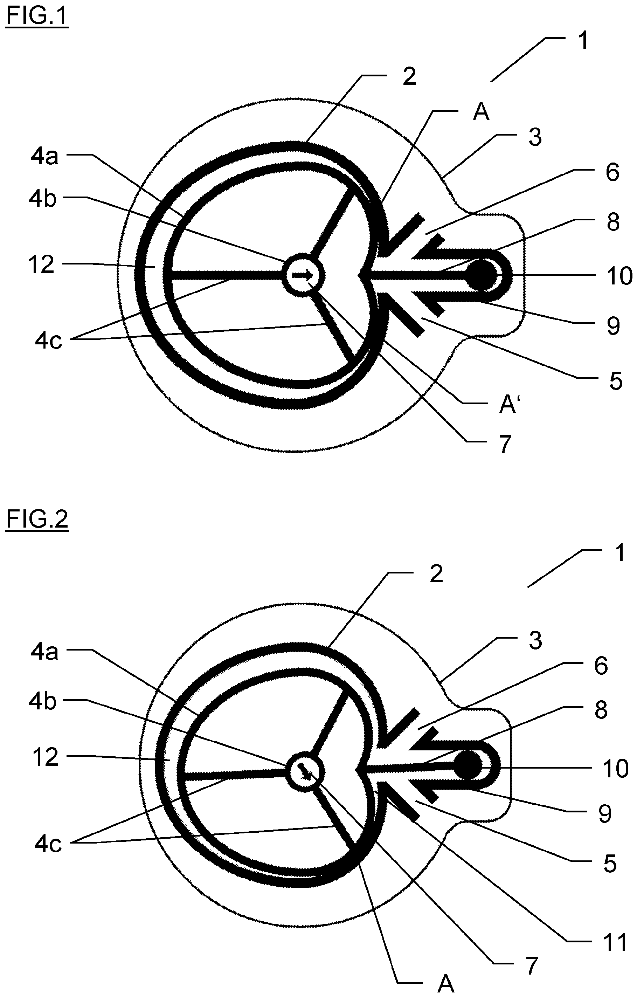

Figur 1 zeigt in einer schematischen Darstellung einen Schnitt durch eine erfindungsgemäße Ausführungsform einer Pumpvorrichtung (1). Die Pumpvorrichtung (1) weist eine Pumpkammer (2) auf, die in ein Gehäuse (3) integriert ist. In der Pumpkammer (2) ist ein Läufer (4) angeordnet. Der Läufer (4) weist eine Läuferwand (4a), einen Läuferkern (4b) und Stege (4c) auf, die den Läuferkern (4b) mit der Läuferwand (4a) verbinden. Durch diese Bauweise ist es möglich, den Läufer (4) im Inneren hohl auszuführen, sodass die Masse des Läufers (4) gering ist. Die Pumpkammer (2) weist einen Einlass (5) und einen Auslass (6) auf, wobei ein Fluid durch den Einlass (5) in die Pumpkammer (2) einströmen und durch den Auslass (6) aus der Pumpkammer (2) ausströmen kann. Der Läufer (4) ist mithilfe eines Antriebs (7) innerhalb der Pumpkammer (2) bewegbar. Der Antrieb (7) ist vorzugsweise als ein Exzenterantrieb ausgebildet, sodass der Läufer (4) in der Pumpkammer (2) auf einer Orbitalbahn bewegbar ist. Auf der rechten Seite weist der Läufer (4) ein als ein Pleuel ausgebildetes Trennelement (8) auf, mit dem der Bereich des Einlasses (5) vom Bereich des Auslasses (6) getrennt ist. An seinem dem übrigen Läufer (4) abgewandten Ende weist das Trennelement (8) ein Dichtelement (10) auf, das in einer Führung (9) linear geführt ist. Mithilfe des Dichtelements (10) ist der Bereich des Einlasses (5) im Bereich der Führung (9) vom Bereich des Auslasses (6) abgedichtet. Durch den Pfeil im Bereich des Antriebs (7) angedeutet ist die Winkelposition des Läufers (4), die hier durch die Nullposition gegeben ist. In der Nullposition bzw. der 0° Winkelposition weist der Läufer (4) zwei Berührungspunkte (A, A') mit der Innenwand der Pumpkammer (2) auf. Der in dieser Position in der Pumpkammer (2) zwischen der Läuferwand (4a) und der Innenwand der Pumpkammer (2) gebildete Druckraum (12) ist durch den ersten Berührungspunkt (A) vom Auslass (6) und durch den zweiten Berührungspunkt (A') vom Einlass (5) getrennt.

Die Formen des Läufers (4) und der Pumpkammer (2) sind derart aufeinander abgestimmt, dass der Verlauf des Volumenstroms bzw. Flows eines aus dem Auslass (6) der Pumpvorrichtung (1) strömenden Fluids bei einer konstanten Geschwindigkeit der Bewegung des Läufers (4) etwa sinusförmig ist. Auf der linken Seite entspricht die Kontur der Läuferwand (4a) etwa der Kontur einer Ellipse mit einem ersten Durchmesser und auf der rechten Seite zweier sich überlappender Kreise mit einem kleineren Durchmesser. Der erste Kreis geht dabei in die beiden kleineren Kreise über.

In Figur 2 ist die in Figur 1 gezeigte Pumpvorrichtung (1) in einer Winkelposition von etwa 60° dargestellt. Der Läufer (4) und die Innenwand der Pumpkammer (2) weisen nur noch einen Berührungspunkt (A) auf. In Bewegungsrichtung, die durch den Uhrzeigersinn gegeben ist, vor dem Berührungspunkt (A) ist zwischen dem Läufer (4) und der Innenwand der Pumpkammer (2) ein Druckraum (12) und in Bewegungsrichtung hinter dem Berührungspunkt (A) ein Saugraum (11) ausgebildet. Durch die Bewegung des Läufers (4) in der Pumpkammer (2) ist ein Fluid über den Einlass (5) in die Pumpkammer (2) hinein saugbar. Das mit dem Läufer (4) verbundene und als ein Pleuel ausgebildete Trennelement (8) ist entsprechend der Position des Läufers (4) etwas geneigt.

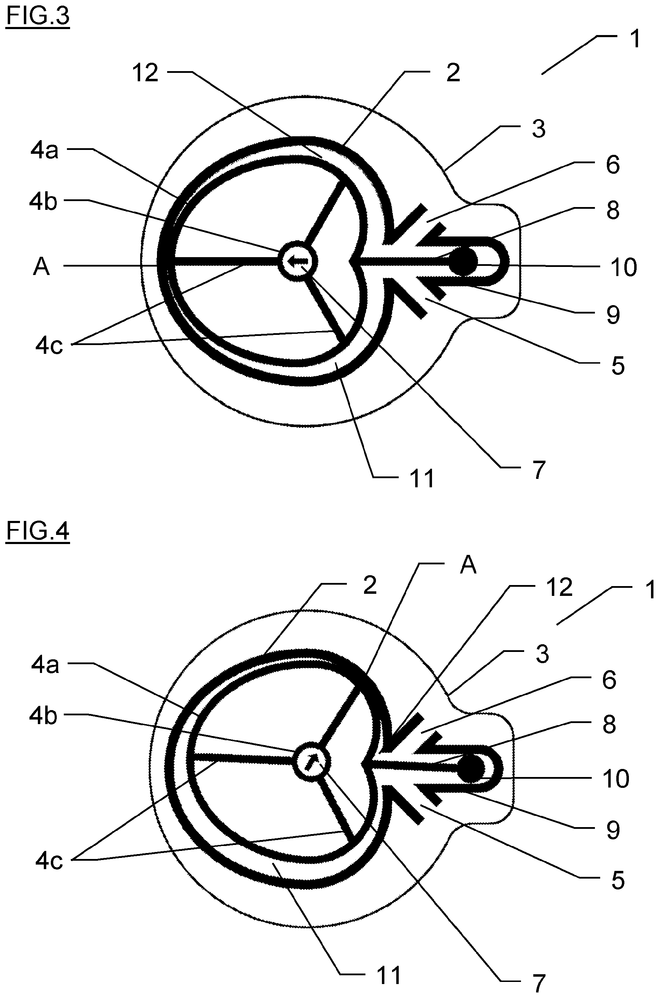

In Figur 3 ist die in den Figuren 1 und 2 dargestellte Pumpvorrichtung (1) in der 180° Winkelposition dargestellt. In dieser Position sind der Saugraum (11) und der Druckraum (12) gleichgroß. Der Volumenstrom eines aus dem Auslass (6) der Pumpvorrichtung (1) strömenden Fluids erreicht in dieser Position sein Maximum.

In Figur 4 ist die in den vorherigen Figuren gezeigte offenbarungsgemäße Pumpvorrichtung (1) in einer Winkelposition von 300° dargestellt. Der Saugraum (11) nimmt nun das überwiegende Volumen der Pumpkammer (2) ein, während der Druckraum (12) nur noch einen sehr geringen Anteil ausmacht. Der Volumenstrom eines aus der Pumpvorrichtung (1) über den Auslass (6) ausströmenden Fluids nähert sich bereits dem Minimum.

Die Form des Läufers (4) und der Pumpkammer (2) sind derart aufeinander abgestimmt, dass nur in der 0° Winkelposition auf der Orbitalbahn des Läufers (4) zwei Berührungspunkte (A, A') zwischen der Läuferwand (4a) und der Innenwand der Pumpkammer (2) gegeben sind, während in allen anderen Winkelpositionen nur ein Berührungspunkt (A) gegeben ist.

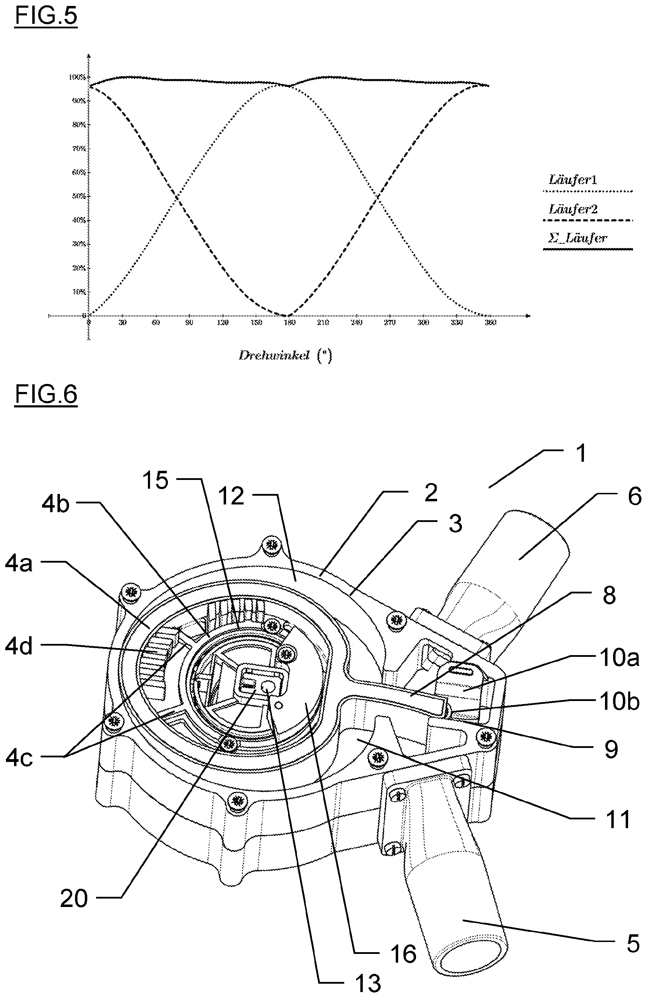

In Figur 5 ist der Flow-Verlauf einer offenbarungsgemäßen Pumpvorrichtung (1) mit zwei Pumpkammern (2) dargestellt, in denen jeweils ein Läufer (4) angeordnet ist, wobei die Läufer (4) zueinander um 180° phasenversetzt angetrieben werden. Der Verlauf des Flows über der Winkelposition entspricht jeweils etwa einem Sinus, wobei je Pumpkammer (2) das Minimum bei 0 % und das Maximum bei mehr als 95 % des Gesamtvolumenstroms liegt. Dargestellt ist weiterhin der kumulierte Flowverlauf (Gesamtvolumenstrom) eines gemeinsamen Auslasses (6) an den beide Pumpkammern (2) angeschlossen sind. Der Gesamtflow der Pumpvorrichtung (1) ist nahezu konstant und variiert nur zwischen etwa 95 % und 100 %.

Figur 6 zeigt eine perspektivische Ansicht einer offenbarungsgemäßen Pumpvorrichtung (1) wobei ein die Pumpkammer (2) nach oben hin abdichtendes Verschlusselement, wie beispielsweise eine abgedichtete Verschlusskappe, nicht montiert ist, sodass die Pumpkammer (2) offen ist. Der Läufer (4) befindet sich etwa in der 180° Winkelposition.

Vom Antrieb (7) ist ein Ende der Welle (13) zu sehen, mit der über eine Verbindungsmechanik (20) eine als ein Wälzlager ausgebildete Entkoppeleinrichtung (15) verbunden ist. Dabei ist die Welle (13) exzentrisch innerhalb der Entkoppeleinrichtung (15) angeordnet, sodass ein Exzenterantrieb des Läufers (4) realisiert ist. Mit der Verbindungsmechanik (20) verbunden ist darüber hinaus ein Massenausgleichselement (16) mit dem die ungleiche Massenverteilung, die ansonsten bei einer Rotation der Welle (13) für eine Unwucht sorgen würde, ausgleichbar ist. Der Läuferkern (4b) ist über Stege (4c) mit der Läuferwand (4a) verbunden. Innen sind im Läufer (4) Rippen (4d) angeordnet, die gemeinsam mit dem Massenausgleichselement (16) dazu dienen, den Schwerpunkt des Läufers (4) in das Zentrum des verwendeten Wälzlagers und/oder in die Mitte der Motorachse bzw. der Welle (13) zu verlagern. Dadurch werden Vibrationen weitestgehend vermieden bzw. zumindest stark unterdrückt. An dem dem Läufer (4) abgewandten Ende des Trennelements (8) ist ein mehrteiliges Dichtelement (10) angeordnet, das ein federndes Widerlager (10a) und eine Laufrolle (10b) aufweist. Durch diese Anordnung ist durch den Druck am Auslass (6) im Zweifel das Trennelement (8) gegen die Gehäusewand auf der gegenüberliegenden Seite drückbar, sodass das Trennelement (8) gegen das Gehäuse abdichtet. Die federbelastete Lauffläche bzw. das federnde Widerlager (10a) sorgt somit für einen Mindestanpressdruck und der Ausgangsdruck hilft im Zweifel darüber hinaus nach.

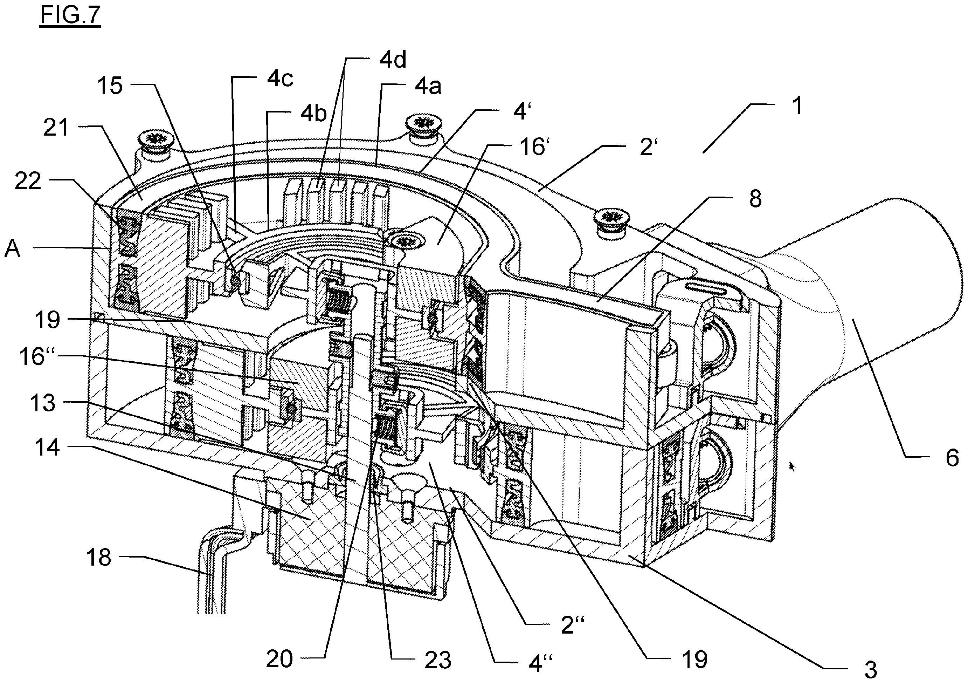

Figur 7 zeigt in einer schematischen Darstellung einen Schnitt in Längsrichtung durch eine erfindungsgemäße Ausführungsform einer Pumpvorrichtung (1) aufweisend zwei Pumpkammern (2', 2") mit jeweils einem zugeordneten Läufer (4', 4"). Der erste Läufer (4') ist in der ersten Pumpkammer (2') angeordnet und befindet sich etwa in der 180° Winkelposition. Der zweite Läufer (4") ist in der zweiten Pumpkammer (2") angeordnet und befindet sich etwa in der 0° Winkelposition. Die erste Pumpkammer (2') ist von der zweiten Pumpkammer (2") durch eine Trennwand (19) getrennt. Weiterhin ist unterhalb der Pumpkammern (2', 2") ein Motor (14) angeordnet, mit dem eine Welle (13) antreibbar ist. Die Welle (13) wird zum Antrieb beider Läufer (4', 4") verwendet. Auf der Welle (13) ist jeweils im Bereich einer Pumpkammer (2', 2") mithilfe einer Verbindungsmechanik (20) eine als ein Kugellager ausgebildete Entkoppeleinrichtung (15) exzentrisch verbunden, sodass die Läufer (4', 4") innerhalb der Pumpkammern (2', 2") auf Orbitalbahnen beweglich sind. Durch die Ausrichtung der Läufer (4', 4") auf der Welle (13) ist der Phasenversatz zwischen den Läufern (4', 4") fest als 180° eingestellt. Durch die Verwendung einer gemeinsamen Welle (13) ist dabei eine Änderung des Phasenversatzes im Betrieb der Pumpvorrichtung (1) ausgeschlossen. Die Massenausgleichselemente (16', 16") sind ebenfalls 180° versetzt zueinander angeordnet.

Die Läufer (4', 4") weisen jeweils an ihren Enden in axialer Richtung einen Gleitring (21) auf, der im Bereich des Trennelements (8) einen Fortsatz aufweist. Die Gleitringe (21) sind dabei jeweils in eine umlaufende Nut eingesetzt, wobei die Gleitringe (21) nicht vollständig in der Nut versenkt sind. Die Gleitringe (21) laufen an den die Pumpkammern (2', 2") in axialer Richtung begrenzenden Wänden und dichten dabei die Pumpkammer (2', 2") gegenüber dem Innenraum des jeweiligen Läufers (4', 4") ab. Zu Verbesserung der Dichtwirkung auch bei erhöhten Bauteiltoleranzen, sind die Gleitringe (21) mit Federelementen (22) derart gefedert, dass diese an die Gehäusewand angedrückt werden. In der dargestellten Ausführungsform der Offenbarung liegen die Federelemente (22) hinter den Gleitringen (21) in der jeweiligen Nut. Die Welle (13) ist mithilfe einer Wellendichtung (23) abgedichtet.

In anderen Ausführungsformen der Offenbarung sind auch andere Positionen des Motors (14) des Antriebs denkbar, beispielsweise zwischen den Pumpkammern (2°, 2").

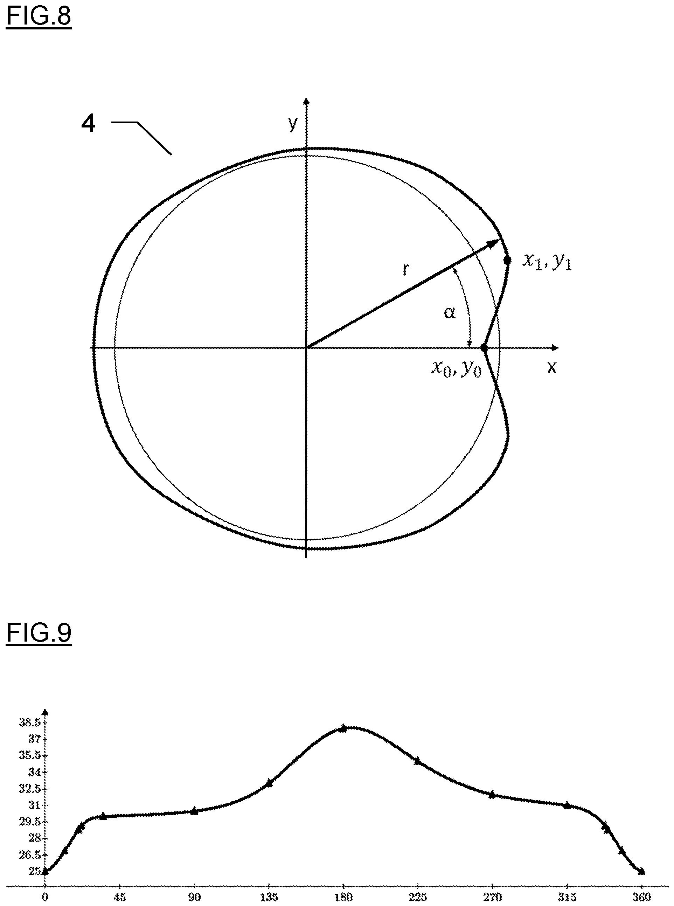

In Figur 8 ist eine schematische Darstellung des Querschnitts eines Läufers (4) einer offenbarungsgemäßen Vorrichtung (1) dargestellt. Die Form des Läufers (4) ist durch eine Spline Funktion definiert. Weiterhin sind der Radius r und der Winkel $\alpha$ angegeben, mit denen die Form des Läufers (4) im dargestellten Koordinatensystem mit X-Achse und Y-Achse beschreibbar ist.

Figur 9 zeigt den Verlauf des Radius r über dem Winkel $\alpha$ des in der Figur 8 dargestellten Läufers.

Die Dreiecke zeigen, welche Werte (Radius über Winkel) für die Spline-Funktion festgelegt wurden (Ziehpunkte). Die Kurve ist eine kubische Spline-Funktion im kartesischen Koordinatensystem, die ins Polarkoordinatensystem übertragen wird.

Die Kurve ist nicht symmetrisch zur 180° Mitte. D.h. dass der Läufer (4) der entsprechenden Ausführungsform einer erfindungsgemäßen Vorrichtung auch nicht symmetrisch zur x-Achse ist. Durch Optimieren der Form ist diese so ausgebildet, dass der Flow des ausströmenden Fluids möglichst konstant ist.

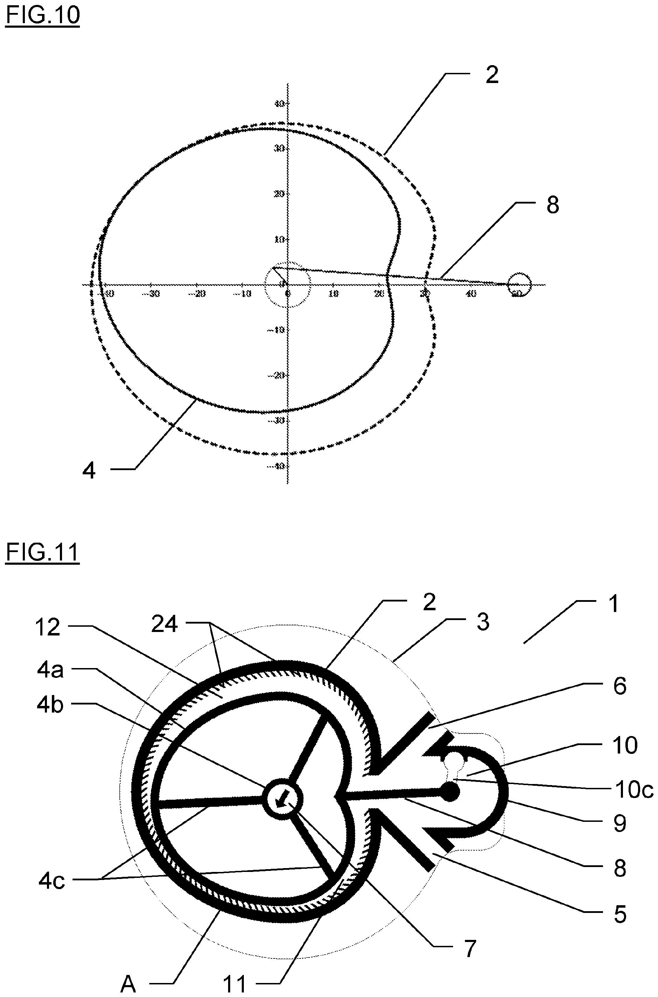

In Figur 10 ist eine schematische Darstellung eines Querschnitts eines entsprechend einer Spline-Funktion geformten Läufers (4) in einer korrespondierend ausgebildeten Pumpkammer (2) dargestellt. Die Kontur der Pumpkammer (2) ergibt sich dabei durch die Einhüllende der Läuferform, die über den Kurbelwinkel entsprechend des Exzenterantriebs über einen vollständigen Umlauf (0° - 360°) verschoben wird.

Der im inneren des Läufers (4) dargestellte Kreis ist der Kreis des Exentermittelpunkts.

Figur 11 zeigt einen Querschnitt einer Ausführungsform einer offenbarungsgemäßen Pumpvorrichtung (1) mit einem Gehäuse (3) der Pumpkammer (2), das federnd gegenüber dem Läufer (4) ausgebildet ist.

Dazu weist das Gehäuse (3) an seiner Innenseite in die Pumpkammer (2) hineinragende Lamellen (24) auf.

Die Lamellen (24) sind in der dargestellten Ausführungsform in Bewegungsrichtung des Läufers (4) leicht schräg angestellt. Dadurch ist eine geringe der Bewegung des Läufers (4) entgegenwirkende Reibungskraft bei gleichzeitig guter Dichtungswirkung realisiert.

Bei einer derartigen Ausführungsform der Erfindung ist je nach Winkelposition des Läufers (4) in der Pumpkammer nicht mehr genau ein Berührungspunkt (A) bzw. sind nicht mehr genau zwei Berührungspunkte, sondern ein Berührungsbereich um diesen Punkt bzw. diese Punkte herum realisiert, in denen der Läufer (4) von einer Mehrzahl von Lamellen (24) kontaktiert wird.

Weiterhin weist die dargestellte Ausführungsform einer erfindungsgemäßen Pumpvorrichtung (1) eine auch für alle anderen gezeigten Ausführungsformen realisierbare Modifikation des Dichtelements (10) auf, das das Trennelement (8) an seiner dem Läufer (4) abgewandten Seite dichtend mit dem Gehäuse (3) der Pumpkammer (2) verbindet. Dabei weist das Dichtelement (10) einen um eine erste Drehachse drehbeweglich am Gehäuse (3) gelagerten Hebel (10c) auf, der mit dem Ende des Trennelements (8) um eine zweite Drehachse drehbeweglich verbunden ist, sodass im Unterschied zur Ausführung als ein dichtend in einer Führung (9) gelagertes Pleuel eine geringere Reibung in diesem Bereich auftritt.

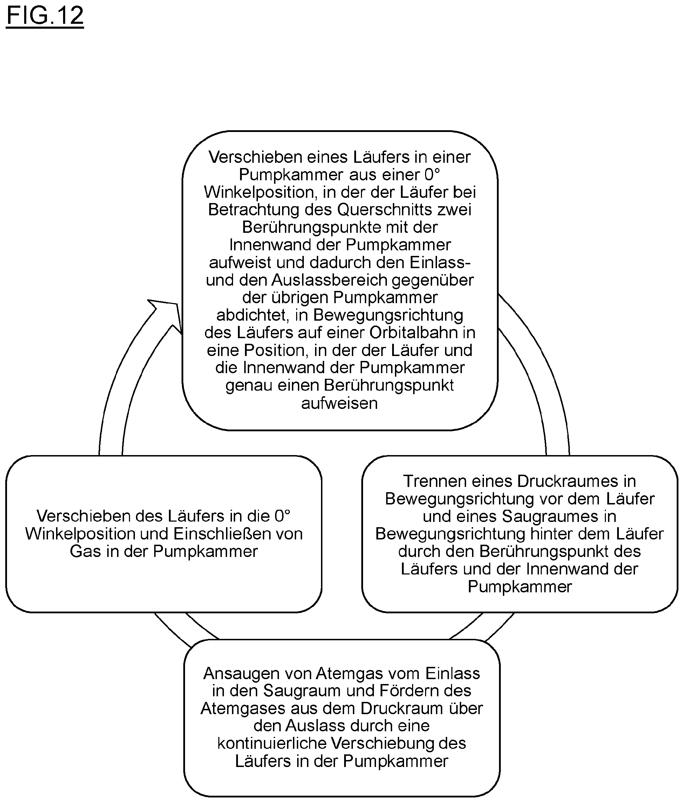

In Figur 12 ist der Ablauf eines offenbarungsgemäßen Verfahrens zur Bereitstellung von Atemgas schematisch dargestellt. Bei der Verwendung einer erfindungsgemäßen Pumpvorrichtung mit einem gegenüber dem Läufer federnden Gehäuse sind anstelle des Berührungspunkts bzw. der Berührungspunkte sich um diese Punkte herum erstreckende Berührungsbereiche zwischen Läufer und Gehäuse realisiert.

**Patentansprüche**

1. Vorrichtung zur Beatmung aufweisend mindestens eine Pumpvorrichtung (1), wobei die Pumpvorrichtung (1) mindestens eine Pumpkammer (2) und je Pumpkammer (2) einen in der Pumpkammer (2) angeordneten Läufer (4) aufweist, wobei die mindestens eine Pumpkammer (2) einen Einlass (5) und einen Auslass (6) aufweist, wobei ein Fluid über den Einlass (5) in die Pumpkammer (2) und über den Auslass (6) aus der Pumpkammer (2) ausströmen kann, wobei der Einlass (5) und der Auslass (6) über einen gemeinsamen Kanal mit der Pumpkammer (2) verbunden sind und wobei der Einlass (5) durch ein in dem Kanal angeordnetes Trennelement (8) vom Auslass getrennt ist und wobei der Läufer (4) innerhalb der Pumpkammer (2) auf einer Orbitalbahn antreibbar ist und die Form des Läufers (4) und die Form der Pumpkammer (2) so ausgebildet und aufeinander abgestimmt sind, dass durch die Bewegung des Läufers (4) in der Pumpkammer (2) mit einer konstanten Geschwindigkeit ein etwa sinusförmiger Verlauf des Flows des aus dem Auslass (6) strömenden Fluids realisiert ist, wobei diese zwei Pumpkammern (2', 2") aufweist und je Pumpkammer (2', 2") einen in der Pumpkammer (2', 2") angeordneten Läufer (4', 4"),

   wobei die Läufer (4', 4") zueinander um 180° phasenversetzt in den Pumpkammern (2', 2") angeordnet sind,
   **dadurch gekennzeichnet, dass** die Pumpkammern (2', 2") einen gemeinsamen Auslass (6) aufweisen, so dass die mithilfe der beiden Pumpkammern (2', 2") und Läufer (4', 4") jeweils erzeugbaren etwa sinusförmigen Flows einen kumulierten, nahezu konstanten Gesamtvolumenstrom ergeben.

2. Vorrichtung zur Beatmung nach Anspruch 1, **dadurch gekennzeichnet, dass** der Läufer (4) durch einen Exzenterantrieb auf der Orbitalbahn beweglich ist.

3. Vorrichtung zur Beatmung nach einem der vorstehenden Ansprüche, **dadurch gekennzeichnet, dass** die Form des Läufers (4) und die Form der Pumpkammer (2) so ausgebildet und aufeinander abgestimmt sind, dass bei der Betrachtung des Querschnitts in der 0° Winkelposition des Läufers (4) innerhalb der Pumpkammer (2) zwei Berührungspunkte (A, A') bzw. zwei um diese Berührungspunkte (A, A') angeordnete Berührungsbereiche zwischen dem Läufer (4) und der Innenwand der Pumpkammer (4) realisiert sind und in allen anderen Winkelpositionen nur ein Berührungspunkt (A) bzw. ein um diesen Berührungspunkt (A) angeordneter Berührungsbereich zwischen dem Läufer (4) und der Innenwand der Pumpkammer (4) realisiert ist.

4. Vorrichtung zur Beatmung nach einem der vorstehenden Ansprüche, **dadurch gekennzeichnet, dass** das Trennelement (8) mit dem Läufer (4) verbunden ist und von diesem aus in den Bereich des Einlasses (5) und des Auslasses (6) ragt.

5. Vorrichtung zur Beatmung nach einem der vorstehenden Ansprüche, **dadurch gekennzeichnet, dass** der Läufer (4) federnd gegenüber dem Gehäuse (3) der Pumpkammer (2) gelagert ist.

6. Vorrichtung zur Beatmung nach einem der vorstehenden Ansprüche, **dadurch gekennzeichnet, dass** das Gehäuse (3) der Pumpkammer (2) federnd gegenüber dem Läufer (4) ausgebildet ist.

7. Vorrichtung zur Beatmung nach Anspruch 6, **dadurch gekennzeichnet, dass** das Gehäuse (3) der Pumpkammer (2) nach innen in die Pumpkammer (2) hineinragende, sich quer zur Bewegungsrichtung des Läufers (4) erstreckende Lamellen (24) aufweist.

8. Vorrichtung zur Beatmung nach einem der vorstehenden Ansprüche, **dadurch gekennzeichnet, dass** die Form des Läufers (4) im Querschnitt entsprechend zu einer in kartesischen Koordinaten generierten und in Polar-Koordinaten übertragenen Spline-Funktion ausgebildet ist, so dass ein Kurvenverlauf ohne Sprünge ermöglicht ist.

9. Vorrichtung zur Beatmung nach einem der Ansprüche 1 bis 8, **dadurch gekennzeichnet, dass** die Läufer (4', 4") mithilfe einer gemeinsamen Welle (13) antreibbar sind.

10. Vorrichtung zur Beatmung nach Anspruch 8, **dadurch gekennzeichnet, dass** die Spline-Funktion aus kubischen Parabeln gebildet ist.

11. Verfahren zur Bereitstellung von Atemgas, aufweisend die Verfahrensschritte Verschieben eines Läufers in einer Pumpkammer aus einer 0° Winkelposition, in der der Läufer zwei Berührungspunkte (A, A') bzw. zwei um diese Berührungspunkte (A. A') angeordnete Berührungsbereiche mit der Innenwand der Pumpkammer aufweist und dadurch den Einlass- und den Auslassbereich gegenüber der übrigen Pumpkammer abdichtet, in Bewegungsrichtung des Läufers auf einer Orbitalbahn in eine Position, in der der Läufer und die Innenwand der Pumpkammer genau einen Berührungspunkt (A) bzw. einen um diesen Berührungspunkt (A) angeordneten Berührungsbereich aufweisen, Trennen eines Druckraumes in Bewegungsrichtung vor dem Läufer und eines Saugraumes in Bewegungsrichtung hinter dem Läufer durch den Berührungspunkt des Läufers und der Innenwand der Pumpkammer, Ansaugen von Atemgas vom Einlass in den Saugraum und Fördern des Atemgases aus dem Druckraum über den Auslass durch eine kontinuierliche Verschiebung des Läufers in der Pumpkammer, wobei mit einem zweiten Läufer in einer zweiten Pumpkammer die gleichen Verfahrensschritte in einem 180° phasenversetzten Ablauf umgesetzt werden, sodass bei einer auf den jeweiligen Orbitalbahnen umlaufenden Bewegung der Läufer innerhalb der jeweiligen Pumpkammer bei einer konstanten Laufgeschwindigkeit an einem gemeinsamen Auslass ein möglichst sprungfreier Flowverlauf des Atemgases realisiert wird, **dadurch gekennzeichnet, dass** eine Vorrichtung zur Beatmung nach einem der Ansprüche 1 bis 10 verwendet wird.

**Claims**

1. Respiratory device comprising at least one pump device (1), wherein the pump device (1) has at least one pump chamber (2) and a rotor (4) arranged in the pump chamber (2) for each pump chamber (2), wherein the at least one pump chamber (2) has an inlet (5) and an outlet (6), wherein a fluid can flow into the pump chamber (2) via the inlet (5) and out of the pump chamber (2) via the outlet (6), wherein the inlet (5) and the outlet (6) are connected to the pump chamber (2) via a common channel and wherein the inlet (5) is separated from the outlet by a separating element (8) arranged in the channel and wherein the rotor (4) can be driven on an orbital path within the pump chamber (2) and the shape of the rotor (4) and the shape of the pump chamber (2) are designed and matched to one another, that by the movement of the rotor (4) in the pump chamber (2) at a constant speed an approximately sinusoidal course of the flow of the fluid flowing out of the outlet (6) is realized, wherein this has two pump chambers (2', 2") and per pump chamber (2', 2") a rotor (4', 4") is arranged in the pump chamber (2', 2"), wherein the rotors (4', 4") are arranged in the pump chambers (2', 2") 180° out of phase with one another, **characterized in that** the pump chambers (2', 2") have a common outlet (6), so that the approximately sinusoidal flows which can be generated with the aid of the two pump chambers (2', 2") and rotors (4', 4") in each case produce a cumulative, virtually constant total volumetric flow.

2. Respiratory device according to claim 1, **characterized in that** the rotor (4) is movable on the orbital path by means of an eccentric drive.

3. Respiratory device according to one of the preceding claims, **characterized in that** the shape of the rotor (4) and the shape of the pump chamber (2) are designed and matched to one another in such a way that, when the cross-section is viewed in the 0° angular position of the rotor (4) inside the pump chamber (2), two points of contact (A, A') or two contact areas arranged around these contact points (A, A') are realized between the rotor (4) and the inner wall of the pump chamber (4) and in all other angular positions only one contact point (A) or one contact area arranged around this contact point (A) is realized between the rotor (4) and the inner wall of the pump chamber (4).

4. Respiratory device according to one of the preceding claims, **characterized in that** the separating element (8) is connected to the rotor (4) and projects from the latter into the region of the inlet (5) and the outlet (6).

5. Respiratory device according to one of the preceding claims, **characterized in that** the rotor (4) is resiliently mounted relative to the housing (3) of the pump chamber (2).

6. Respiratory device according to one of the preceding claims, **characterized in that** the housing (3) of the pump chamber (2) is designed to be resilient with respect to the rotor (4).

7. Respiratory device according to claim 6, **characterized in that** the housing (3) of the pump chamber (2) has lamellae (24) projecting inwards into the pump chamber (2) and extending transversely to the direction of movement of the rotor (4).

8. Respiratory device according to one of the preceding claims, **characterized in that** the shape of the rotor (4) is designed in cross-section corresponding to a spline function generated in cartesian coordinates and transferred to polar coordinates, so that a curve progression without jumps is made possible.

9. Respiratory device according to one of claims 1 to 8, **characterized in that** the rotors (4', 4") can be driven by means of a common shaft (13).

10. Respiratory device according to claim 8, **characterized in that** the spline function is formed from cubic parabolas.

11. Method for providing a respiratory gas, comprising the method steps of displacing a rotor in a pump chamber from a 0° angular position, in which the rotor has two contact points (A, A') or two contact regions arranged around these contact points (A. A') with the inner wall of the pump chamber and thereby seals the inlet and outlet areas from the rest of the pump chamber, in the direction of movement of the rotor on an orbital path to a position in which the rotor and the inner wall of the pump chamber have exactly one point of contact (A) or a point of contact (A') arranged around these points of contact (A. A'). an area of contact arranged around this point of contact (A), separating a pressure chamber in the direction of movement in front of the rotor and a suction chamber in the direction of movement behind the rotor by the point of contact of the rotor and the inner wall of the pump chamber, drawing in breathing gas from the inlet into the suction chamber and conveying the breathing gas from the pressure chamber via the outlet by a continuous displacement of the rotor in the pump chamber, wherein the same process steps are implemented in a 180° phase-shifted sequence with a second rotor in a second pump chamber, so that with a movement of the rotors rotating on the respective orbital paths within the respective pump chamber at a constant running speed at a common outlet, a flow course of the respiratory gas which is as jump-free as possible is realized, **characterized in that** a respiratory device according to one of claims 1 to 10 is used.

## Revendications

1. Dispositif de ventilation présentant au moins un dispositif de pompage (1), le dispositif de pompage (1) présentant au moins une chambre de pompage (2) et pour chaque chambre de pompage (2) un rotor (4) agencé dans la chambre de pompage (2), l'au moins une chambre de pompage (2) présentant une entrée (5) et une sortie (6), un fluide pouvant s'écouler par l'entrée (5) dans la chambre de pompage (2) et par la sortie (6) hors de la chambre de pompage (2), l'entrée (5) et la sortie (6) étant reliées à la chambre de pompage (2) par un canal commun, et l'entrée (5) étant séparée de la sortie par un élément de séparation (8) agencé dans le canal, et le rotor (4) pouvant être entraîné à l'intérieur de la chambre de pompage (2) sur une trajectoire orbitale, et la forme du rotor (4) et la forme de la chambre de pompage (2) étant configurées et adaptées l'une à l'autre de telle sorte que le mouvement du rotor (4) dans la chambre de pompage (2) à une vitesse constante permet de réaliser une allure approximativement sinusoïdale du flux du fluide s'écoulant hors de la sortie (6), celui-ci présentant deux chambres de pompage (2', 2") et, pour chaque chambre de pompage (2', 2"), un rotor (4', 4'') agencé dans la chambre de pompage (2', 2''), les rotors (4', 4'') étant agencés déphasés de 180° l'un par rapport à l'autre dans les chambres de pompage (2', 2''), **caractérisé en ce que** les chambres de pompage (2', 2'') présentent une sortie commune (6), de telle sorte que les flux approximativement sinusoïdaux pouvant être générés à l'aide des deux chambres de pompage (2', 2'') et rotors (4', 4'') donnent un débit volumique total cumulé presque constant.

2. Dispositif de ventilation selon la revendication 1, **caractérisé en ce que** le rotor (4) est mobile sur la trajectoire orbitale grâce à un entraînement excentrique.

3. Dispositif de ventilation selon l'une quelconque des revendications précédentes, **caractérisé en ce que** la forme du rotor (4) et la forme de la chambre de pompage (2) sont configurées et adaptées l'une à l'autre de telle sorte qu'en observant la section transversale dans la position angulaire de 0° du rotor (4) à l'intérieur de la chambre de pompage (2), deux points de contact (A, A') ou deux zones de contact agencées autour de ces points de contact (A, A') sont réalisés entre le rotor (4) et la paroi intérieure de la chambre de pompage (4) et, dans toutes les autres positions angulaires, un seul point de contact (A) ou une seule zone de contact agencée autour de ce point de contact (A) est réalisé entre le rotor (4) et la paroi intérieure de la chambre de pompage (4).

4. Dispositif de ventilation selon l'une quelconque des revendications précédentes, **caractérisé en ce que** l'élément de séparation (8) est relié au rotor (4) et fait saillie à partir de celui-ci dans la zone de l'entrée (5) et de la sortie (6).

5. Dispositif de ventilation selon l'une quelconque des revendications précédentes, **caractérisé en ce que** le rotor (4) est monté élastiquement par rapport au boîtier (3) de la chambre de pompage (2).

6. Dispositif de ventilation selon l'une quelconque des revendications précédentes, **caractérisé en ce que** le boîtier (3) de la chambre de pompage (2) est configuré sous forme élastique par rapport au rotor (4).

7. Dispositif de ventilation selon la revendication 6, **caractérisé en ce que** le boîtier (3) de la chambre de pompage (2) présente des lamelles (24) faisant saillie vers l'intérieur dans la chambre de pompage (2), s'étendant transversalement à la direction de déplacement du rotor (4).

8. Dispositif de ventilation selon l'une quelconque des revendications précédentes, **caractérisé en ce que** la forme du rotor (4) en section transversale est configurée pour correspondre à une fonction spline générée en coordonnées cartésiennes et transposée en coordonnées polaires, de manière à permettre une allure de courbe sans sauts.

9. Dispositif de ventilation selon l'une quelconque des revendications 1 à 8, **caractérisé en ce que** les rotors (4', 4") peuvent être entraînés à l'aide d'un arbre commun (13).

10. Dispositif de ventilation selon la revendication 8, **caractérisé en ce que** la fonction spline est formée de paraboles cubiques.

11. Procédé de fourniture de gaz respiratoire, présentant les étapes de procédé de déplacement d'un rotor dans une chambre de pompage à partir d'une position angulaire de 0°, dans laquelle le rotor présente deux points de contact (A, A') ou deux zones de contact agencées autour de ces points de contact (A, A') avec la paroi intérieure de la chambre de pompage et étanchéifie ainsi la zone d'entrée et la zone de sortie par rapport au reste de la chambre de pompage, dans la direction de déplacement du rotor sur une trajectoire orbitale, jusqu'à une position dans laquelle le rotor et la paroi intérieure de la chambre de pompage présentent exactement un point de contact (A) ou une zone de contact agencée autour de ce point de contact (A), la séparation d'un espace de pression avant le rotor dans la direction de déplacement et d'un espace d'aspiration après le rotor dans la direction de déplacement par le point de contact du rotor et de la paroi intérieure de la chambre de pompage, l'aspiration de gaz respiratoire de l'entrée dans l'espace d'aspiration et le refoulement du gaz respiratoire de l'espace de pression via la sortie par un déplacement continu du rotor dans la chambre de pompage, les mêmes étapes de procédé étant mises en œuvre avec un deuxième rotor dans une deuxième chambre de pompage dans un déroulement déphasé de 180°, de telle sorte que, lors d'un mouvement de circulation des rotors sur les trajectoires orbitales respectives à l'intérieur de la chambre de pompage respective à une vitesse de marche constante, une allure de flux du gaz respiratoire aussi exempte de sauts que possible est réalisée à une sortie commune, **caractérisé en ce qu'**un dispositif de ventilation selon l'une quelconque des revendications 1 à 10 est utilisé.

FIG.1

FIG.2

FIG.3

FIG.4

## FIG.5

Läufer1

Läufer2

Σ_Läufer

Drehwinkel (°)

## FIG.6

FIG.7

EP 4 175 704 B1

FIG.8

FIG.9

## FIG.10

## FIG.11

## FIG.12

Verschieben eines Läufers in einer Pumpkammer aus einer 0° Winkelposition, in der der Läufer bei Betrachtung des Querschnitts zwei Berührungspunkte mit der Innenwand der Pumpkammer aufweist und dadurch den Einlass- und den Auslassbereich gegenüber der übrigen Pumpkammer abdichtet, in Bewegungsrichtung des Läufers auf einer Orbitalbahn in eine Position, in der der Läufer und die Innenwand der Pumpkammer genau einen Berührungspunkt aufweisen

Verschieben des Läufers in die 0° Winkelposition und Einschließen von Gas in der Pumpkammer

Trennen eines Druckraumes in Bewegungsrichtung vor dem Läufer und eines Saugraumes in Bewegungsrichtung hinter dem Läufer durch den Berührungspunkt des Läufers und der Innenwand der Pumpkammer

Ansaugen von Atemgas vom Einlass in den Saugraum und Fördern des Atemgases aus dem Druckraum über den Auslass durch eine kontinuierliche Verschiebung des Läufers in der Pumpkammer